# EUROPEAN PATENT APPLICATION

(11) **EP 2 266 589 A1**
(43) Date of publication of application: **29.12.2010**
(21) Application number: 09162801.6
(22) Date of filing: 16.06.2009
(51) Int. Cl.: A61K 36/074, A61P 9/10

(54) **Ganoderma tsugae active substance having endothelial cell-protecting and atherosclerosis-preventing effects, process for preparing the same and composition containing the same**

(71) Applicant: Syngen biotech CO., LTD., Sinying City (TW)
(72) Inventor: Wei, Yu-Shan, Houbi Shiang (TW); Hsieh, Chia-Wen, Chiayi City (TW); Wung, Being-Sun, Taipei City (TW)
(74) Representative: Viering, Jentschura & Partner

(57) **Abstract**

A *Ganoderma tsugae* active substance, produced from *Ganoderma tsugae* by following steps: (1) liquid culturing *Ganoderma tsugae*; (2) extracting the fermentation filtrate obtained with ethanol to obtain ethanol extraction precipitate; (3) purifying the ethanol extraction precipitate obtained by gel filtration chromatography to obtain gel filtration chromatographed product; and (4) extracting the gel filtration chromatographed product obtained with acetone to obtain the supernatant as the desired *Ganoderma tsugae* active substance. The invention provides further a composition comprising *Ganoderma tsugae* active substance produced by the above-described steps, said composition is useful for inducing or increasing the quantity of intranuclear transcription factor Nrf2, promoting the activities of antioxidant response element promoter (ARE promoter) and Thioredoxin reductases promoter (TrxR promoter), and inducing expressions of Heme oxygenase-1 (HO-1) and Thioredoxin reductases (TrxR), so as to achieve effects of protecting cell or preventing atherosclerosis.

## Description

### BACKGROUND OF THE INVENTION

### 1. FIELD OF THE INVENTION

The invention relates to *Ganoderma tsugae* active substance, and in particular, to *Ganoderma tsugae* active substance capable of protecting endothelial cell and preventing atherosclerosis.

### 2. DESCRIPTION OF THE PRIOR ART

For several thousand years, in Oriental countries, plants of the Ganodermataceae family have proved to have medicinal effects. For example, antioxidant effect is one of their medicinal effects. *Ganoderma tsugae (Ganoderma tsugae)* is a flat polypore mushroom of the genus Ganoderma, and is also a cultivar used as a dietary supplement in Taiwan. In recent years, extracts from fruit body and mycelium obtained by solid culturing of *Ganoderma tsugae* have been reported to have medicinal effects such as immunoregulation, anti-cancer, anti-inflammation, antioxidant effect and the like. However, there has been little understanding of the antioxidant properties of exopolysaccharides or other active substances produced from the liquid fermentation culturing of *Ganoderma tsugae.*

Oxidative stress plays an important role in the pathophysiology of atherogenesis. Oxidative stress can be defined as the pathogenic outcome of the overproduction of oxidants that overwhelm the antioxidant capacities of soluble antioxidants and free radical-quenching enzymes. Thus control over the activities of these enzymes may protect against free radical damage. Recent studies have demonstrated that Heme oxygenase-1 (HO-1) is cytoprotective due to its antioxidant activities. Overexpressed HO-1 reduced atherogenesis, which highlighted the potential of HO-1 as a novel therapeutic target for the prevention of cardiovascular diseases. More recently, a redox-sensitive transcription factor, NF-E2-related factor-2 (Nrf2), was shown to mediate expression of many antioxidant proteins, including HO-1, glutathione S-transferase, glutamylcysteine synthetase and thioredoxin reductase (TrxRl). In this respect, the search for novel and more potent inducers of Nrf-2 related genes will facilitate the development of new therapies for the prevention or treatment of cardiovascular disorders.

In view of the extensive effects and the pharmacological studies on atherosclerosis of Ganoderma described above, the inventors had devoted to study the effect of active substance produced from the liquid culturing of *Ganoderma tsugae* on the expressions of Heme oxygenase-1 (HO-1) and Thioredoxin reductases (TrxR1) in endothelial cell, and successfully found *Ganoderma tsugae* active substance having endothelial cell-protecting and atherosclerosis- preventing effects according to the invention.

### SUMMARY OF THE INVENTION

The object of the invention is to provide *Ganoderma tsugae* active substance having endothelial cell-protecting and atherosclerosis-preventing effects.

Another object of the invention is to provide a process for preparing said *Ganoderma tsugae* active substance, said process comprising treating *Ganoderma tsugae* mycelium through following steps:
step 1: culturing *Ganoderma tsugae* to obtain culture thereof;
step 2: press filtering the culture obtained in step 1, and extracting the filtrate with ethanol to obtain ethanol extraction precipitate;
step 3: purifying the ethanol extraction precipitate by gel filtration chromatography to obtain gel filtration chromatographed products;
step 4: extracting the gel filtration chromatographed products obtained in step 3 with acetone to obtain the supernatant as the desired *Ganoderma tsugae* active substance.

The culturing manner mentioned in step 1 includes, but not limited to, liquid fermentation culturing, solid state culturing or other culturing method suitable to the invention.

In a preferred embodiment, *Ganoderma tsugae* used is *Ganoderma tsugae* CGMCC 2861 deposited in China General Microbiological Culture Collection Center (CGMCC), on January 8, 2009. Nonetheless, *Ganoderma tsugae* active substance described in the invention is not limited to those obtained from this strain.

In a preferred embodiment, said liquid fermentation culturing comprises the following steps: culturing in a culturing liquor containing 0.1-1% of protein hydrolysate, 0.2-2% of complex nitrogen source, 0.01-0.1% of trace minerals, 0.01-0.1% of inorganic salts and 2-10% of complex carbon source, under conditions of 20 - 35 °C, tank pressure of 0.5-1 vvm, with stirring rate of 20-100 rpm for 5-15 days; wherein said complex nitrogen source may use cereals or beans; wherein said inorganic salts may include magnesium sulfate, dipotassium hydrogen phosphate, iron sulfate and the like; and wherein said complex carbon source may include glucose, sucrose, fructose, maltose and the like.

In a preferred embodiment, said ethanol extraction step comprises: extracting with ethanol having final concentration of 10% for 30-60 minutes, centrifuging, and extracting the supernatant thus obtained with ethanol having final concentration of 20% for 30-60 minutes. In another preferred embodiment, said ethanol extraction step comprises extracting with ethanol having final concentration of 20% for 30-60 minutes.

In a preferred embodiment, said gel filtration chromatography comprises the following step: loading the ethanol extraction precipitate on a Sephacryl S-400 gel column, and eluting with 0.05-0.5 M tris(hydroxymethyl)aminomethane (Tris) buffer solution (pH 6.8-7.2) as the mobile phase at a flow rate of 0.5 ml/min, and collecting fractions of those in the interval of 161-200 ml or 321-400 minutes.

In a preferred embodiment, said acetone extraction step comprises of extracting with acetone having final concentration of 55% at 8--20 °C for 30-120 minutes.

Still another object of the invention is to provide a composition containing said *Ganoderma tsugae* active substance, and suitable diluents, excipients or carriers, **characterized in that** said composition is capable of protecting endothelial cell and preventing atherosclerosis.

These features and advantages of the present invention will be fully understood and appreciated from the following detailed description of the accompanying Drawings.

### BRIEF DESCRIPTION OF THE DRAWINGS

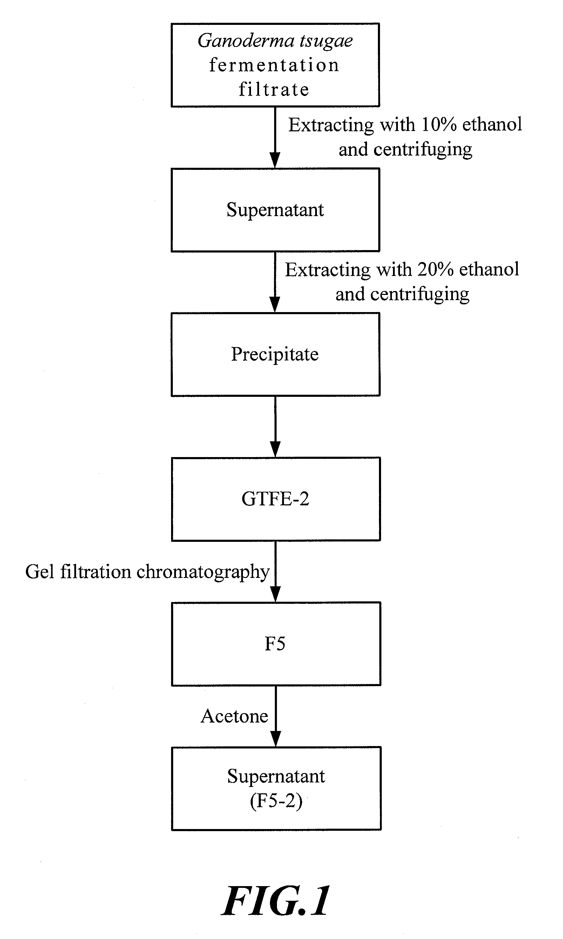

Figure 1 is a flow chart for extracting *Ganoderma tsugae* active substance according to the invention, showing steps for preparing different extracts from liquid culturing suspension of *Ganoderma tsugae.*

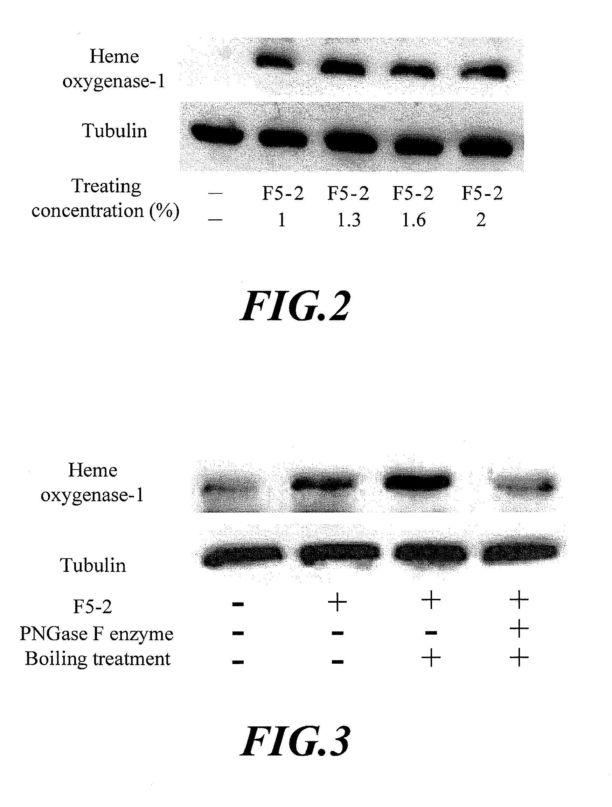

Figure 2 shows the analysis on expression of Heme oxygenase-1(HO-1) in endothelial cell induced by *Ganoderma tsugae* extract F5-2, wherein "-" represents a control group (same treatment but without adding extract).
Figure 3 shows the analysis that revealed the weakened induction on the expression of Heme oxygenase-1(HO-1) by the PNGase F enzyme hydrolyzed *Ganoderma tsugae* extract F-2; wherein "-" represents that the treatment had not been carried out, and "+" represents that said treatment had been carried out; for example, the first lane indicates that F5-2 had not been treated by endothelial cell, that F5-2 had not been hydrolyzed by PNGase F enzyme and not subjected to boiling treatment.

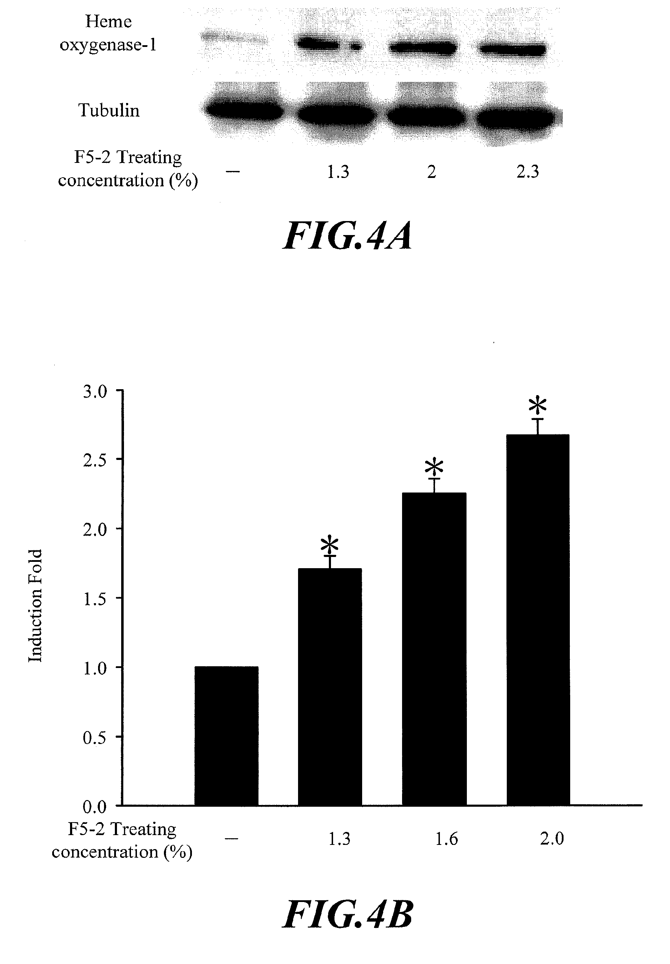

Figure 4A shows the analysis on the expression of Thioredoxin reductases (TrxR-1) induced by *Ganoderma tsugae* extract F-2. Figure 4B shows the analysis on promoter activity of Thioredoxin reductases (TrxR) induced by *Ganoderma tsugae* extract F-2; wherein "-" represents the control group (same treatment but without adding extract).

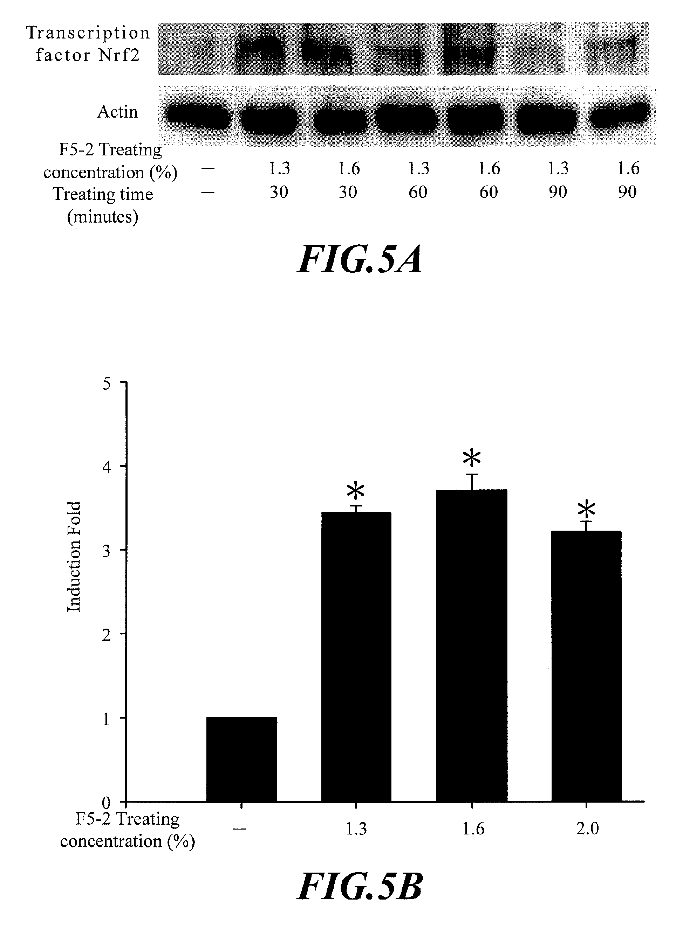

Figure 5A and 5B show respectively the increase of translocation of intra-nuclear transcription factor Nrf2 and the induction of ARE-luciferase reporter activity expression caused by *Ganoderma tsugae* extract F-2; wherein "-" represents the control group (same treatment but without adding extract).

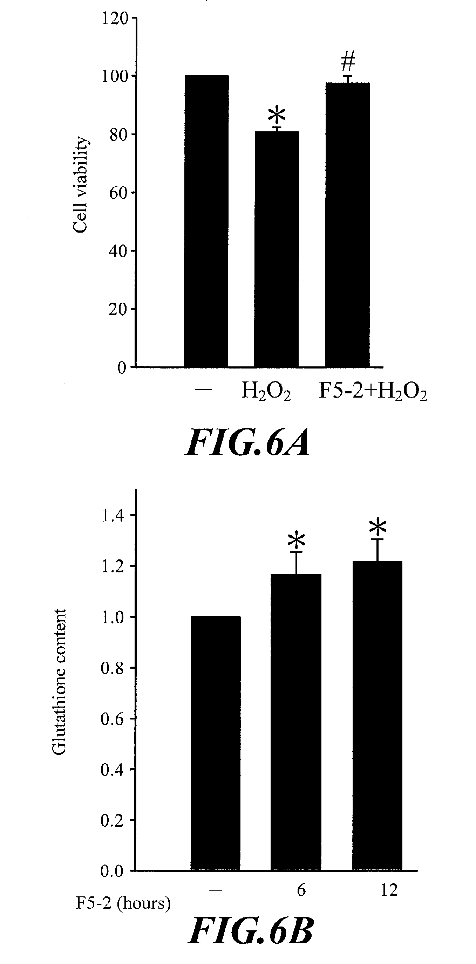

Figure 6A shows the analysis of the protection effect of *Ganoderma tsugae* extract F-2 against oxidative stress. The result is expressed as mean ± SEM. Said test was repeated independently more than five times; *p <0.05 means relative to the untreated control group, and indicates that there is a significant difference therebetween; #p<0.05 means relative to the H₂O₂-treated only control group, and indicates that there is a significant difference therebetween; Figure 6B shows the analysis of expression of glutathione (GSH) by *Ganoderma tsugae* extract F-2; wherein "-: represents the control group (same treatment but without adding extract).

### DETAILED DESCRIPTION OF THE PREFERRED EMBODIMENT

The invention will be illustrated by way following examples, but the invention is not limited to the following examples.

### Example 1 The preparation of Ganoderma tsugae fermentation liquor

### 1.1 The source of Ganoderma tsugae

*Ganoderma tsugae* used in this example was obtained from *Ganoderma tsugae* CGMCC 2861 deposited in China General Microbiological Culture Collection Center (CGMCC), on January 8, 2009. Nonetheless, *Ganoderma tsugae* active substance described in the invention is not limited to those obtained from this strain.

### 1.2 Culturing of Ganoderma tsugae:

*Ganoderma tsugae* was inoculated on Potato Dextrose Agar (PDA, Difco™, REF213400) in a Petri dish. After cultured at 20-35°C for 5-10 days, the agar was cut into fragments and was inoculated into Potato Dextrose Broth (PDB, Difco™, REF254920) in a 3L concave shaking flask, and cultured under conditions of 20-35°C and shaking rate of 100-200 rpm for 5-10 days.

*Ganoderma tsugae* seed culture obtained in the 3L concave shaking flask was transferred in a sterile accepting flask on a sterile operation bench, and then transferred through a steam-sterilized pipeline into a 50L fermentation seed culturing tank containing nutritional sources of 0.1-1% of protein hydrolysate, 0.2-2% of complex nitrogen source, 0.01-0.1% of trace minerals, 0.01-0.1% of inorganic salts, 2-10% of complex carbon source. Fermentation was started under conditions of pH 4-6, 20-35°C, tank pressure 0.5-1vvm (sterile air), and stirring rate of 90-120 rpm for 3-7 days.

Thereafter, the seed culture in the 50L fermenter was transferred through a sterile pipeline that had been steam sterilized for 2 hours into a 500L fermentation seed culturing tank containing nutritional sources of 0.1-1% of protein hydrolysate, 0.2-2% of complex nitrogen source, 0.01-0.1% of trace minerals, 0.01-0.1 % of inorganic salts, 2-10% of complex carbon source, and fermentation was started under conditions of pH 4-6, 20-35°C, tank pressure 0.5-1vvm (sterile air), and stirring rate of 20-50 rpm for 3-7 days.

Then, the seed culture in the 500L fermenter was transferred through a sterile pipeline that had been steam sterilized for 2 hours into a 5000L fermentation seed culturing tank containing nutritional sources of 0.1-1% of protein hydrolysate, 0.2-2% of complex nitrogen source, 0.01-0.1% of trace minerals, 0.01-0.1% of inorganic salts, 2-10% of complex carbon source, and fermentation was started under conditions of pH 4-6, 20-35°C, tank pressure 0.5-lvvm (sterile air), and stirring rate of 20-60 rpm for 5-10 days.

The *Ganoderma tsugae* fermentation liquor was press filtered as followed: Celite was added at an amount of 5-10% into the 5000L fermenter, mixed homogeneously with said *Ganoderma tsugae* mycelium fermentation liquor with stirring, and then transferred through sterile pipeline into a platen press and press filtration was performed to obtain *Ganoderma tsugae* fermentation filtrate.

### Example 2 Preparation of Ganoderma tsugae active substance and analysis of ingredients and biological activities

### 2.1 Material

HO-1 primary antibody was obtained from Stressgen Biotechnologies (SB, San Diego, CA). Antibodies against Nrf2 were purchased from Santa Cruz Biotechnology (Santa Cruz, CA, USA). Peroxidase-conjugated anti-rabbit and anti-mouse antibodies were purchased from Amersham (Arlington Heights, IL, USA). All other reagents, materials were purchased from Sigma (St. Louis, MO) or other available commercial product.

### 2.2 Method

### (1) Endothelial cell cultures

Bovine aortic endothelial cells (BAECs) were cultured in Dulbecco's modified Eagle's medium (DMEM, Invitrogen) supplemented with 10% fetal bovine serum (FBS; Invitrogen) and suitable antibiotics. Cells were kept at 37°C in a humidified atmosphere of air and 5% CO₂. Cells were grown in Petri dishes and allowed to reach confluence. The culture medium was then replaced with serum free DMEM and the cells were incubated for 12 hours prior to experimental treatments.

### (2) Preparation of cell lysates

The whole lysates of endothelial cell was obtained according a conventional technique for obtaining whole lysates. To prepare nuclear extracts, endothelial cells were collected by scraping in cold PBS. The cell pellet was then lysed in 10 mM HEPES, 1.5 mM MgCl₂, 10 mM KCl, 0.5 mM DTT, 0.5 mM PMSF and 0.3% Nonidet P-40. Nuclear proteins were then extracted using a buffer containing 25% glycerol, 20 mM HEPES, 0.6 M KCl, 1.5 mM MgCl₂, and 0.2 mM EDTA. Protein concentrations were determined using a protein assay DC system (Bio-Rad, Richmond, CA, USA).

### (3) Western blotting

Western blotting was carried out in accordance with conventional technical procedure, briefly described as follows:
A total of 1 × 10⁶ cells were incubated on ice in lysis buffer (1% NP-40, 0.5% sodium deoxycholate, 0.1% SDS and a protease inhibitor mixture) and the resulting whole-cell extracts were separated on 10% SDS-PAGE. The proteins were then transferred to a nitrocellulose filter (Millipore). Membranes were incubated with HO-1 antibodies for two hours at 4°C, with gentle shaking. The results were visualized by chemiluminescence using ECL reagents (Amersham Pharmacia Biotech), according to the manufacturer's instructions.

### (4) Cell Viability Assay

Cell viability was evaluated using an Alamar blue assay according to the manufacturer's instructions (Serotec, Oxford, UK). This assay is based on the detection of metabolic activity in living cells using a redox indicator that changes from an oxidized (blue) to a reduced (red) form. The intensity of the red color is proportional to the viability of cells and is calculated as the difference in absorbance between 570 and 600 nm. The values are expressed as a percentage of the control.

### (5) Transient transfections and luciferase assays

One day prior to transfection, ECs were subcultured at a density of 1 × 10⁶ cells in dishes to obtain approximately confluent cultures. The cells were transiently transfected using lipofectamine with a plasmid containing ARE sequences, according to the instructions given by the manufacturer (GIBCO-BRL). The cells were co-transfected in each case with a pSV-β-galactosidase plasmid to normalize for transfection efficiency. After overnight transfection, the cells were then treated with various concentrations of F5-2, washed with PBS and lysed with the reporter lysis buffer supplied for luciferase assays (Promega). Lysates were centrifuged and supernatants thus obtained were added with NPG (ortho-nitrophenyl-β-D-galactopyranoside), mixed well and stood still for 2-3 days. An ELISA reader was used to read values (A) under a wavelength of 420 nm. Separately, to other supernatant aliquots, luciferase activity assay agents were added and mixed, luciferase activity values (B) in term of reporter expression were read immediately in a Luminometer. Ratio of the two values was calculated (B/A) and then divided by the value of the control group. Results thus obtained were depicted in a bar chart.

### (6) GSH Assay

Cell culture was treated with F5-2, liquid medium was aspirated off, and the residue was washed with phosphate buffered saline (PBS). Cells were treated with monochlorobimane (MCB, 40 µM) under light protection. Cells were lysed, and relative GSH content was determined under exciting wavelength of 380 nm and emitting wavelength of 470nm in a fluorescence spectrometer (Shimadzu, Rf-5301PC).

### (7) Bioassay-guided isolation of F5-2 from G tsugae

*Ganoderma tsugae* fermentation filtrate was extracted in accordance with the flow chart depicted in Fig. 1. The freeze-dried *Ganoderma tsugae* fermentation filtrate was dissolved in water, extracted with 10%, and 20% ethanol, and centrifuged to obtain precipitate. The precipitate was dried in vacuum at 40°C, dissolved in de-ionized water, and labeled as GTFE-2.

GTFE-2 was subjected to gel filtration chromatography to separate ingredients of GTFE-2 under following conditions: a Sephacryl™S-400 column (16 mm × 100 cm) (GE Healthcare, Little Chalfont, Buckinghamshire, USA) was used, and eluted with 0.05-0.5 M Tris buffer solution (pH 6.8-7.2) as the mobile phase with a flow rate of 0.5 mL/min. 4 mL aliquots were collected, and fractions of those in the interval of 161-200 ml or 321-400 minutes were combined and referred as F5.

F5 was extracted with acetone and centrifuged. Acetone in the supernatant was removed under vacuum at 40°C. The thus-obtained isolation solution was referred as F5-2. F5-2 was then subjected to chemical analysis and biological activity assay.

### Characterization of F5-2

### (8) Glycolysis of F5-2 with PNGase F enzyme, and analysis of the ability for inducing the expression of Heme oxygenase-1 (HO-1)

To the F5-2 extract, 0.5 M sodium phosphate (pH 7.5), 50 mM EDTA, 0.5% (w/v) SDS and 5% (v/v) β-mercaptoethanol were added, and glycolysis was conducted with peptide-N-glycosidase F (PNGase F, BioLabs^{@}Inc., New England) at 37°C for 12 hours. 1.6 % of F5-2 was used to act on bovine aortic endothelial cell (BAECs) for 6 hours. In addition, boiled F5-2 or PNGase F glycolyzed F5-2 was used to treat cells. Cell lysates were collected and Western blotting assay was conducted against Heme oxygenase-1 (HO-1) antibody or tubulin (used as internal control).

### (9) Statistical analysis

Data for all experiments were obtained from at least three independent experiments, each performed in triplicate. These data were then analyzed by analysis of variance (ANOVA), and subsequently, for significant ANOVAs, pair-wise comparisons between the control group and treatment groups were done by post hoc comparison using the Tukey test (SPSS software package, Chicago, IL, USA). *p*<0.05 was considered as indicating a statistically significant difference between groups. In all graphs, data are shown as mean ± SD.

### 2.3 Results

### (1) Expression of Heme oxygenase-1(HO-1) induced by Ganoderma tsugae active substance F5-2

F5-2 was assayed by Western blotting to determine the expression quantity of Heme oxygenase-1 (HO-1) in endothelial cell. Bovine aortic endothelial cell (BAECs) was treated with extract F-2 (1, 1.3, 1.6 or 2.0 %), respectively, for 6 hours. Western blotting was used to determine the expression quantity of HO-1 protein in the cell using tubulin as the internal control. Result shown in Fig. 2 indicates that F5-2 could induce the expression of Heme oxygenase-1 (HO-1) significantly.

### (2) Enzymatic hydrolysis of F5-2 with PNGase F (a glycoprotein hydrolytic enzyme) and weakening of the ability for inducing the expression of Heme oxygenase-1 (HO-1)

PNGase F enzyme acts primarily on the bonding site of GlcNAc and asparagines in glycoprotein or glycopeptides having N-glycan. F5-2 was subjected to enzymatic hydrolysis by PNGase F, and cellular HO-1 activity was determined. Result shown in Fig. 3 indicates that the induction activity of PNGase F-treated F5-2 was weakened. Accordingly, the activity of F5-2 for inducing the expression of Heme oxygenase-1 (HO-1) in endothelial cell might come from N-glycans.

### (3) F5-2: a peptidoglycan analogue

Total carbohydrate and protein contents in F5-2 were determined, wherein total carbohydrate was determined by phenol-sulfuric acid method, and protein concentration was determined by Lowry-Folin method. Results were: total carbohydrate content 63.7 mg/mL, protein content, 0.77 mg/mL, and ratio of total carbohydrate to protein content was 82 to 1. Amino acids in F5-2 were analyzed and 16 types of different amino acids were detected. Said amino acids and concentration (mg/mL) thereof were: asparagine (Asp) 0.83, threonine (Thr) 0.44, serine (Ser) 0.41, glutamic acid (Glu) 1.63, proline (Pro) 0.54, glycine (Gly) 0.46, alanine (Ala) 0.70, cystine (Cys) 0.17, valine (Val) 0.43, methionine (Met) 0.10, isoleucine (Ile) 0.31, leucine (Leu) 0.44, tyrosine (Tyr) 0.10, phenylalanine (Phe) 0.26, lysine (Lys) 0.20, histidine (His) 0.36, and arginine (Arg) 0.35. F5-2 was hydrolyzed with trifluoroacetic acid (TFA), and mole ratio of various monosaccharides was determined by gas chromatography. F5-2 contained six types of monosaccharides: D-glucose, D-mannose), N-acetyl glucosamine, L-arabinose, D-galactose, and L-fucose. Mole ratio of these monosaccharides was 12.8: 3.2: 5.2: 5.2: 1.2: 1. Further, F5-2 displayed protein band on SDS-PAGE by Coomassie blue staining analysis, and displayed red polysaccharide band on SDS-PAGE by Schiff's reagent staining analysis. Molecular weight of F5-2 was 11 KDa as determined by gel permeation chromatography (GPC). These results indicate that F5-2 is a peptidoglycan analogue.

### (4) Induction of TrxR-1 expression by F5-2

In endothelial cell treated with F5-2, it could be found that the expression of Heme oxygenase-1 (HO-1) was increased. Therefore, F5-2 was analyzed further to determine whether it could induce the expression of other gene in relation to transcription factor Nrf2. As shown in Fig. 4A, When endothelial cell was treated with F5-2's having concentrations of 1.3%, 2.0%, 2.3%, respectively, the protein expression of thioredoxin reductase (TrxR-1) (another gene in relation with transcription factor Nrf2) was increased also. Cell was transfected with a construct bearing TrxR-luciferase (conventional TrxR promoter ligated with reporter luciferase on the construct). After 12 hours, the thus-transfected cell was cultured with low-serum medium for 6 hours. Then, cell was treated with F5-2 having different concentrations (1.3, 1.6, 2.0 %) for 12 hours. Thereafter, cell was lysed and the luciferase activity was assayed; the result was shown in Fig. 4B. It could be seen that the promoter activity of thioredoxin reductases (TrxR) could be activated by F5-2 treatment.

### (5) F5-2 increases the rate of Nrf2 translocation and induces ARE-luciferase reporter activity

We further examined whether Nrf2 itself is activated by F5-2 treatment in endothelial cells (ECs). As shown in Figure 5A, ECs treated with 1.3% and 1.6% F5-2 showed a higher level of Nrf2 accumulation in the nuclear fraction compared with untreated cells, as early as 30 minutes after initiation of treatment.

Previous studies have identified Nrf2 as a major transcription factor that regulates antioxidant response element (ARE) driven gene expression. The specificity of F5-2 for this ARE sequence was then determined by transfecting endothelial cells with luciferase reporter constructs harboring this element. As shown in Figure 5B, cells treated with F5-2 displayed an increase in ARE-luciferase activity.

### (6) Protective effects of F5-2 against oxidative stress

Present invention indicated that F5-2 is a potent inducer of HO-1 and TrxR-1 expression and we therefore wanted to test whether it had a protective role in endothelial cells (ECs) against oxidative stress. For this purpose, cells were initially pretreated with 1.6% F5-2 for 6 hours to facilitate Nrf-2-related gene induction. The medium was then replaced with 10% hydrogen peroxide for two hours followed by an assessment of cell viability. As shown in Figure 6A, exposure of control ECs to hydrogen peroxide resulted in a substantial loss in cell viability but pretreatment of these cells with F5-2 significantly attenuated this cytotoxicity.

GSH is an anti-oxidant and increased by induction of Nrf-2-dependent enzyme, glutamylcysteine synthetase. We therefore tested GSH levels over the time course of F5-2 pretreatment. As shown in Figure 6B, GSH levels increased after 6 hours of F5-2 pretreatment and the increase persisted for over 12 hours. This data suggests that F5-2-induced genes are required to counteract oxidative stress.

*Ganoderma tsugae* active substance provided by the invention have following advantages over conventional techniques or substance:
Experiments demonstrated that by increasing the level of intranuclear transcription factor Nrf 2 (promoting the translocation of Nfr2 into cell nucleus), *Ganoderma tsugae* active substance provided according to the invention can increase the activities of antioxidant response element promoter (ARE promoter), and of TrxR promoter, as well as induces further expressions of Heme oxygenase-1 (HO-1) and thioredoxin reductases (TrxR), so as to achieve the effect of preventing atherosclerosis; under comparison, conventional *Ganoderma tsugae* active substance do not have such effects.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A *Ganoderma tsugae* active substance, produced from *Ganoderma tsugae* by following steps:
step 1: culturing *Ganoderma tsugae* to obtain culture thereof;
step 2: extracting the *Ganoderma tsugae* culture with ethanol to obtain ethanol extraction precipitate;
step 3: purifying the ethanol extraction precipitate obtained in step 2 by gel filtration chromatography to obtain gel filtration chromatographed product;
step 4: extracting the gel filtration chromatographed product obtained in step 3 with acetone to obtain the supernatant as the desired *Ganoderma tsugae* active substance.

2. Ganoderma tsugae active substance as in claim 1, wherein said Ganoderma tsugae strain is *Ganoderma tsugae* CGMCC 2861 deposited in China General Microbiological Culture Collection Center (CGMCC), but said *Ganoderma tsugae* active substance is not limited to those obtained from this strain.

3. Ganoderma tsugae active substance as in claim 1, wherein said culturing step comprises: culturing in a culturing liquor containing 0.1-1% of protein hydrolysate, 0.2-2% of complex nitrogen source, 0.01-0.1% of trace minerals, 0.01-0.1% of inorganic salts and 2-10% of complex carbon source, under conditions of 20-35°C, tank pressure of 0.5-1 vvm, with stirring rate of 20-100 rpm for 5-15 days.

4. Ganoderma tsugae active substance as in claim 1, wherein said ethanol extraction step comprises: extracting with ethanol having final concentration of 10% for 30-60 minutes, centrifuging, and extracting the supernatant thus obtained with ethanol having final concentration of 20% for 30-60 minutes.

5. Ganoderma tsugae active substance as in claim 1, wherein said ethanol extraction step comprises: extracting with ethanol having final concentration of 20% for 30-60 minutes.

6. Ganoderma tsugae active substance as in claim 1, wherein said gel filtration chromatography step comprises: loading said ethanol extraction precipitate on a gel column, and eluting with a salt buffer solution as the mobile phase at a flow rate of 0.5 ml/min, and collecting fractions of those in the interval of 161-200 ml or 321-400 minutes.

7. Ganoderma tsugae active substance as in claim 6, wherein said gel column is Sephacryl S-400 column.

8. Ganoderma tsugae active substance as in claim 6, wherein said salt buffer solution is 0.05-0.5 M tris(hydroxymethyl)aminomethane (Tris) buffer solution (pH 6.8-7.2).

9. Ganoderma tsugae active substance as in claim 1, wherein said acetone extraction step comprises of extracting with acetone having final concentration of 55% at 8--20°C for 30-120 minutes.

10. A composition for preventing atherosclerosis, comprising *Ganoderma tsugae* active substance as recited in claim 1, and suitable diluent, excipient, or carrier.

11. Composition as in claim 10, for inducing the activity of Heme oxygenase-1.

12. Composition as in claim 10, for inducing the activity of thioredoxin reductases.

13. Composition as in claim 10, for promoting the translocation of transcription factor Nfr2 into cell nucleus.
